# EUROPEAN PATENT SPECIFICATION

(11) **EP 0 560 407 B1**
(45) Date of publication and mention of the grant of the patent: **22.05.1996**
(21) Application number: 93107518.8
(22) Date of filing: 09.12.1987
(51) Int. Cl.: C07D 233/60, C07D 277/36, C07D 257/04, C07D 213/70, C07D 249/08, C07D 239/54, C07D 233/64, C07D 235/26, C07D 233/91, C07D 213/30, C07D 413/12, C07D 235/06, C07D 473/00, C07D 233/90, A61K 31/41

(54) **Pharmaceutically active compounds**
Pharmazeutisch wirksame Verbindungen
Composés pharmaceutiquement actifs

(30) Priority: 10.12.1986 US 940125
(43) Date of publication of application: 15.09.1993
(62) Divisional of application: 87310807.0
(73) Proprietor: SCHERING CORPORATION, Kenilworth New Jersey 07033 (US)
(72) Inventor: Girijavallabhan, Viyyoor Moopil, Parsippany, New Jersey 07054 (US); Ganguly, Ashit Kumar, Upper Montclain, New Jersey 07043 (US); Pinto, Patrick Anthony c/o Schering-Plough Corp., Madison, New Jersey 07940-1000 (US); Versace, Richard William, Wanaque, New Jersey 07465 (US)
(74) Representative: Ritter, Stephen David

(56) References cited:
- EP-A- 0 049 060
- EP-A- 0 051 827
- EP-A- 0 086 043
- EP-A- 0 112 292
- EP-A- 0 210 753
- EP-A- 0 218 543
- US-A- 3 190 888
- US-A- 4 540 703

## Description

This invention relates to compounds with pharmaceutical activity, i.e. antiviral activity, their pharmaceutically acceptable salts and solvates and pharmaceutical compositions containing the active compounds.

Arildone, a compound represented by the formula 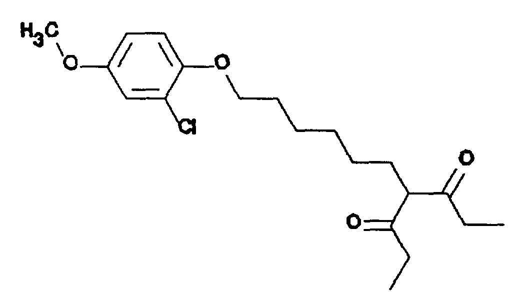 is active in vitro against herpes virus and polio virus, but is only marginally active against rhinoviruses. Diana et al., J. Med. Chem. 28, 748 (1985) prepared some alkyl-substituted isoxazole analogs of arildone in an attempt to prepare compounds with broad spectrum activity against picornaviruses. Some of the compounds were active against both rhinovirus type 2 and poliovirus type 2.

Other antiviral agents are known in the art. US-A-3190888 describes aryloxyalkylpyrazole compounds having anti-viral activity. EP-A-0112292 describes compounds having growth regulatory and microbicide activity containing terminal azolyl groups.

Other pharmaceutical compositions are described in AU-A-56398/86, having activity as lipogenase inhibitors, anti-inflammatory agents and anti-allergic agents.

It is an object of the present invention to provide anti-viral compounds.

The invention relates broadly to compounds of I and II. Compounds of formula I are the subject of European patent 0274867, granted on an application from which the subject matter described and claimed herein has been divided out. Compounds of formula I have the following structural formula:-

Z-X-Q-Y-W

The compounds of this invention are represented by the following structural formula II

Z-X-Q-Y-W'-Y-Q-X-Z II

pharmaceutically acceptable acid addition, basic addition, and quaternary amine salts thereof and pharmaceutically acceptable solvates thereof, wherein
each Z is independently tertiary butyl, phenyl, naphthyl or adamantanyl; substituted phenyl, wherein the substituents are one or more of halogen, lower alkoxy, phenoxy, nitrile, nitro, phenylsulfonyl, loweralkylsulfonyl, oxazol-2-yl, lower alkanoyl, benzoyl, lower alkoxycarbonyl, lower alkyl, phenyl, lower alkylthio, phenylaminothiocarbonyl, or lower alkylaminothiocarbonyl;
X and Y are each independently a bond, -O-, 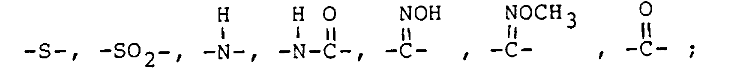
each Q is independently a divalent substituted or unsubstituted, straight or branched chain lower alkanediyl, loweralkanediyl-cycloalkanediylloweralkanediyl, lower alkenediyl, lower alkynediyl, phenylene, dihydrofurandiyl, tetrahydrofurandiyl, tetrahydropyrandiyl, loweralkanediyl-tetrahydrofurandiylloweralkanediyl wherein the substituents are one or more of epoxy, fluorine, chlorine, azide, or amino;
W is a monovalent substituted or unsubstituted aryl group or a heterocyclic single or fused ring containing from 4 to 10 ring atoms, at least one hetero atom of which is a nitrogen atom and the remaining ring atoms being at least one carbon and optionally sulfur or oxygen, wherein the substituents are one or more of hydroxy, oxo, amino, carbamoyl, carboxyl, nitrile, nitro, lower alkyl, loweralkoxycarbonyl, halogen, sulfamyl, loweralkoxycarbonylloweralkyl, loweralkythio, lower alkoxy, hydroxy loweralkyl, amino loweralkyl, carboxy loweralkyl, guanidino, thioureido, lower alkyl sulfonylamino, aminocarbonylloweralkyl, allyloxycarbonylmethyl or carbamoyloxyloweralkyl, with the proviso that W cannot be substituted or unsubstituted isoxazolyl,
W' is divalent W.

The invention also includes pharmaceutical compositions containing pharmaceutically effective amounts of a compound of formula II as well as method of treating virus infections using the appropriate pharmaceutical compositions.

As used herein "lower alkyl" alone or in combined form, e.g. "lower alkoxy" or "loweralkanediyl", means straight or branched chain alkyl groups of from 1 to 10 carbon atoms, e.g. methyl, ethyl, propyl, isopropyl, butyl, t-butyl, pentyl, neopentyl, hexyl and the like.

The heterocyclics at Z and W are monovalently bonded to X and Y respectively by a hetero atom, preferably nitrogen, or by a carbon atom. The heterocyclics at W' preferably have two nitrogens, each of which is bonded to a separate -Y-Q-X-Z moiety.

Heterocyclic groups within the scope of this invention for Z are, for example, imidazolyl (such as imidazol-l-yl, imidazol-2-yl, imidazol-4-yl, and imidazol-5-yl), dihydrothiazolyl (such as 4, 5-dihydrothiazol-2-yl), tetrazol (such as tetrazol-5-yl, tetrazol-1-yl, and tetrazol-2-yl), pyridinyl (such as pyridin-2-yl), triazolyl (such as 1, 2, 4-triazol-1-yl), tetrahydro-pyrimidinyl (such as 1, 2, 3, 4-tetrahydro-pyrimidin-1-yl), dihydro-oxazolyl (such as 4, 5-dihydro-oxazol-2-yl), pyrrolidinyl (such as pyrrolidin-1-yl), pyrazolyl (such as pyrazol-1-yl and pyrazol-2-yl), morpholinyl, and azetidinyl. All possible attachment positions of the above heterocyclic groups are within the scope of this invention.

Heterocyclic groups within the scope of this invention for W are, for example, all those listed above for Z and, in addition, fused ring compounds, for example benzamidazolyl (such as benzamidazol-1-yl and benzamidazol-2-yl), naphthyridinyl (such as naphthyridin-1-yl), purine (such as purine-9-yl and purine-7-yl), and quinolinyl.

Heterocyclic groups within the scope of this invention for W' are all those listed above for Z and W, but being divalent. For example, if W' were tetrahydro-pyrimidinyl, it could be 1, 2, 3, 4-tetrahydro-pyrimidin-1, 3-diyl. W' as a benzamidazolyl group could be, for example benzamidazol-1,3-diyl. In other words, to obtain a possible W' group from the groups listed for Z and W, the "yl" suffix in the Z and W radical is replaced by "diyl".

"Aryl" as used herein refers to phenyl and naphthyl.

"Halogen" as used herein means chlorine, fluorine, bromine or iodine with chlorine or fluorine preferred.

"Cycloalkane", alone or in combined form, means a 4, 5, 6 or 7 membered saturated carbocyclic ring.

"Lower alkene", alone or in combined form, means a 2 to 10 carbon branched or straight chain alkene group.

"Lower alkyne", alone or in combined form, means a 2-10 carbon branched or straight chain alkyne group.

"Pharmaceutically acceptable salts" as used herein means acid addition salts formed from mineral acids such as hydrochloric, hydrobromic, phosphoric or sulfuric acids, or formed from organic carboxylic or sulfonic acids such as trifluoroacetic, para-toluene, sulfonic, maleic, acetic, citric, oxalic, succinic, benzoic, tartaric, fumaric, mandelic, ascorbic and malic acids, or quaternary salts prepared from such organic halides as methyl iodide, ethyl iodide, benzyl chloride and the like, although all pharmaceutically acceptable quaternary salts are contemplated. Basic addition salts are also within the scope of this invention.

The above salts are made by conventional means in the art, e.g. reaction of the compound with the appropriate acid, organic halide, or base.

The preferred salt is the hydrochloride salt.

"Hydroxy protecting group" as used herein means any known hydroxy protecting group which is removed by conventional reactions which do not adversely affect the compounds produced. Typical suitable hydroxy protecting groups are t-butyldimethylsilyl (TBDMS) or tetrahydro-pyranyl.

The compounds of this invention have been found to be active against ether-resistant RNA viruses, i.e. picornaviruses which includes enteroviruses and rhinoviruses. The enteroviruses include poliovirus, coxsackieviruses and echoviruses. Rhinoviruses include those viruses associated with the common cold and certain other respiratory ailments. Over one hundred serotypes are identified. Although the compounds of this invention are not active against all the rhinoviruses, they are active against a large number of them including rhinovirus 2. The compounds of this invention are also active against the enteroviruses such as poliovirus 2, coxsackieviruses A and B3, ECHO and hepatitis A.

In addition, the compounds of this invention are active against certain DNA viruses such as herpesvirus and cytomegalovirus. Thus, they showed activity when tested in in vitro activity assays, i.e. plaque reduction assays which measure the ability of synthetic compounds to neutralize virus infectivity, e.g. picornavirus infectivity. In tests against coxsackievirus 3, the IC₅₀ values of the tested compounds of this invention varied from about 0.7 microgram/ml to about 1.4 microgram/ml. The IC₅₀ value in all antiviral tests is the concentration of test compound in micrograms per milliliter which results in a 50% decrease in plaque forming units compared to a non-treated control.

In a modified standard test, i.e. wherein the virus and test compound are mixed and incubated prior to overlaying with an agar medium, active compounds of this invention had IC₅₀s of from about 6.0 to about 37.3 against poliovirus 2, about 8.5 to about 39.5 against human rhinovirus 14 and about 2.4 to 5.0 against cosackievirus B3.

The standard test involves overlaying HeLa cells with agar medium containing measured concentrations of the test compound following virus absorption, then incubating for 72 hours. The resulting plaques are stained, visualized and measured to determine virus growth inhibition as evidenced by plaque reduction when compared to a control.

The modified standard test is considered more sensitive because of its ability to discriminate more clearly the virus growth neutralizing effects between compounds whose IC₅₀s are very close according to the standard test.

Of the antiviral compounds within the scope of formula II, those which form water soluble acid addition salts are orally absorbable, show good tissue levels and serum stability.

The compounds of this invention are conventionally formulated for oral, parenteral, topical and transdermal use, oral is preferred.

This invention includes within its scope pharmaceutical compositions comprising the compounds of this invention in admixture with a pharmaceutically acceptable carrier therefor. In addition, the present invention also includes the use of the compounds of formula II for preparing pharmaceutical compositions useful for treating viral infections. In the foregoing compositions, the active compounds of this invention can be used alone as the sole active antiviral agent, or in combination with other therapeutic agents.

For the preferred oral administration, the compounds of this invention are typically formulated in the form of tablets, capsules, elixirs, solutions, suspensions and the like preferably solutions. For parenteral administration, they may be formulated into solutions or suspensions. Topical formulations such as lotions, creams, ointments, sprays and mechanical delivery devices, e.g. transdermal can also be made with the compounds of this invention.

Typical pharmaceutically acceptable carriers for use in the formulations described above are exemplified by: sugars such as lactose, starches such as corn starch, cellulose and derivatives such as sodium carboxymethyl cellulose, ethyl cellulose and methyl cellulose; and other carriers well known in the art. The compositions may also contain preservatives, aerosol propellants and coloring, thickening, suspending, dispensing, emulsifying, wetting, stabilizing and buffering agents.

The dosage of the compounds of this invention which is administered is dependent, in the judgment of the attending clinician, upon a variety of factors, e.g. the age and weight of the individual being treated, the mode of administration, the potency of the administered compound, the indication for which the drug is administered and the severity of the ailment being treated.

Typically, the dosage administered per day for treating viral infections will be oral administration of from about 1 mg/kg to about 75 mg/kg daily in single or divided doses, with about 1-25 mg/kg preferred.

The compounds of this invention are prepared by the following methods:
(A) to produce a compound of formula I, a compound of the formula

   Z-X-Q'-L¹

   wherein Z and X are as defined previously,
   Q' is the same as Q defined previously, or, provided Q in formula I is to contain at least one of the group 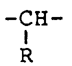 wherein each R is independently hydrogen or lower alkyl, Q' may also be the same as Q defined above minus at least one of the groups 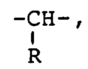 and
   L¹ is a leaving group, with a compound having the formula

      L-Y'-W"

      where L is a leaving group,
   W" is as defined for W in formula I, or a tautomer thereof, and
   Y' is the same Y defined in formula I, or, provided Q in formula I is to contain at least one of the groups 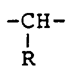 wherein each R is independently hydrogen or lower alkyl, Y' may also be the same as Y defined in formula I plus at least one of the groups 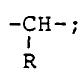 or
(B) to produce a compound of formula II and possibly a compound of formula I, at least one compound of the formula

   Z-X-Q'-L¹

   wherein Z, X, Q' and L¹ are as defined previously is reacted with a compound of the formula

   L³-Y'-W'''-Y'-L⁴

   wherein L³ and L⁴ are leaving groups
   each Y' is independently as defined above, and
   W''' is divalent W' as defined above, or
   (C) to produce a compound of formula I wherein Z and W are the same and X and Y are the same, reacting a compound of the formula

      L-Y'-W"

      wherein Y' and W" are as defined previously and
   L is a leaving group, with a compound of the formula

      L⁵-Q"-L⁶

      wherein L⁵ and L⁶ are leaving groups and Q" is divalent Q' as defined above,
   wherein in the above processes, any reactive groups are protected if necessary or desired,
   the above processes followed, if necessary or desired. by
   (i) removal of any protecting groups,
   (ii) conversion of a compound so produced to another compound of formula I or formula II,
   (iii) if more than one compound of formulas I or II is produced, separation of the compounds so produced, or
   (iv) conversion of any of the compounds so produced to an acid addition, basic addition, or quaternary amine salt or pharmaceutically acceptable solvate thereof.

In process (A), L¹ is preferably bromine or most preferably iodine and L is a preferably alkali metal such as sodium, potassium or cesium. The reaction takes place at temperatures of from about -20°C to 60°C in an inert organic solvent such as dimethylsulfoxide (DMSO), dimethylformamide (DMF) or tetrahydrofuran (THF). In most cases the final compounds can be converted to water-soluble acid addition or quaternary salts by conventional reactions, e.g., with hydrochloric acid or a quaternizing agent such as methyl sulfonic acid.

The starting compounds Z-X-Q'-L¹ wherein L¹ is halogen (Hal) are prepared by the following reaction: 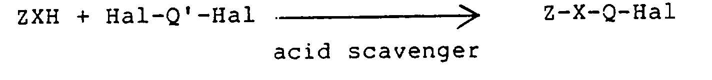 wherein Z, X and Q' are as defined above.

The reaction takes place in the presence of an acid scavenger such as K₂CO₃ or organic bases such as collidine and also Hunigs base. The preferred Hal group is iodine although bromine can also be used. The compound Hal-Q'-Hal wherein Hal is iodine can be prepared by reacting Br-Q'-Br with sodium iodide except when Q is -CH₂- or -CH₂-CH₂-.

Alternatively ZXQ'-I can be prepared by reacting ZXH with Br-Q'-Br to obtain ZXQ'Br, then reacting ZXQ'Br with sodium iodide to obtain Z-X-Q'-I. Or for compounds when Q' is -CH₂- or -C₂H₄-, Z-X-Q'-I can be prepared by reacting the corresponding mono or dihydric alcohol with HI.

When W is a nitrogen containing heterocyclic moiety, the processes described above result in the W moiety being substituted at a nitrogen atom of the heterocyclic ring, unless the nitrogen atom is protected. In order to make a compound wherein the heterocyclic is substituted at a ring carbon, it is necessary to protect the nitrogen with a group which is easily removed after the C-substitution is carried out and is not removed during the C-substitution reaction, e.g., the trityl group.

Thus, for example, in the preparation of a 2-substituted imidazole, the following reaction scheme is followed: 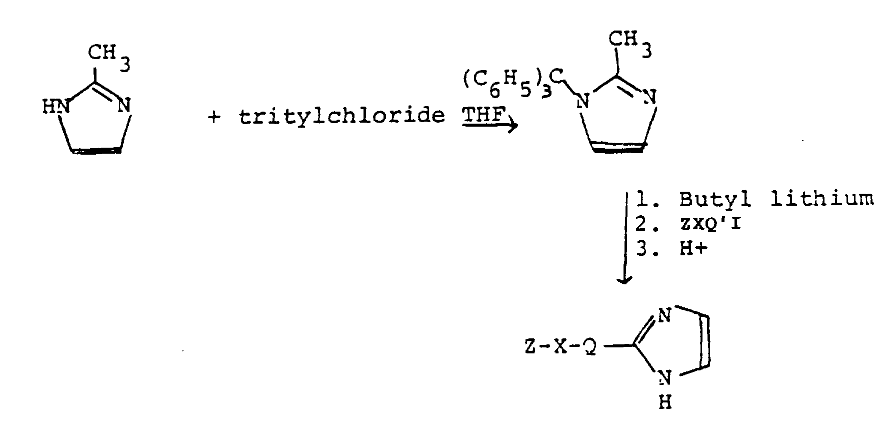 wherein Q, Q', Z and X are as defined above. In this case the methyl group on the imidazo ring becomes part of Q in the compound produced.

In process (B) to make a compound of formula II, the W' moiety should have two ring carbons initially substituted with trimethylsilyloxy groups. When the reaction with Z-X-Q'-I is conducted, the resulting product is a disubstituted W' moiety as shown in the following reaction in which the trimethylsilyloxy substituted compound is illustrated as a pyrimidine.

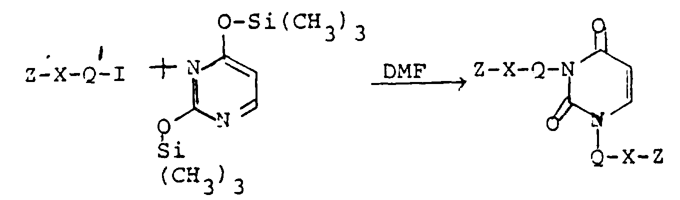

The reaction is carried out at room temperature.

In process (B) if an excess of the compound Z-X-Q'-L¹ is used, the product will primarily be of formula II. However, if only small amounts of the compound Z-X-Q'-L¹ is used, products of formulas I and II will be produced. The reaction conditions are the same as in process (A).

To produce a compound of formula II wherein at least one of Z, X or Q is not identical with the other Z, X or Q two different compounds having the general formula Z-X-Q'-L¹ are used. The result is a mixture of compounds, which may be isolated by standard techniques.

Process C, which produces compounds of formula I wherein Z and W are the same and X and Y are the same, is carried out under the same reaction conditions as process (A).

The preparation of compounds wherein Q is an unsaturated chain, i.e. an alkynediyl or alkenediyl, is illustrated in the following reaction: 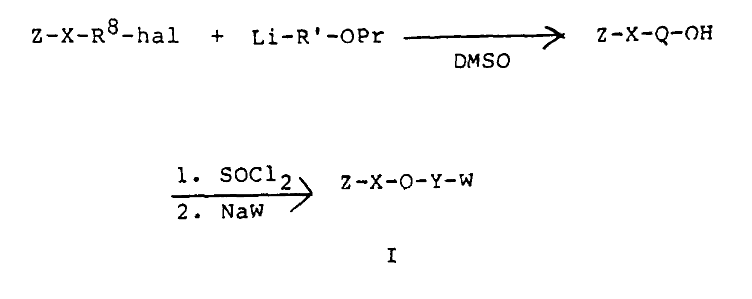 wherein R⁸ is lower alkyl of 2 to 4 carbons and R' is lower alkynyl of 3 carbons, Y is a bond, Pr is a hydroxy protecting group and hal is bromine or iodine.

The alkenediyl is prepared by partially reducing the alkynediyl compound by catalytic hydrogenation.

The preparation of compounds in which Q is an alkyl having a cycloalkane in the chain is illustrated by the following reaction: 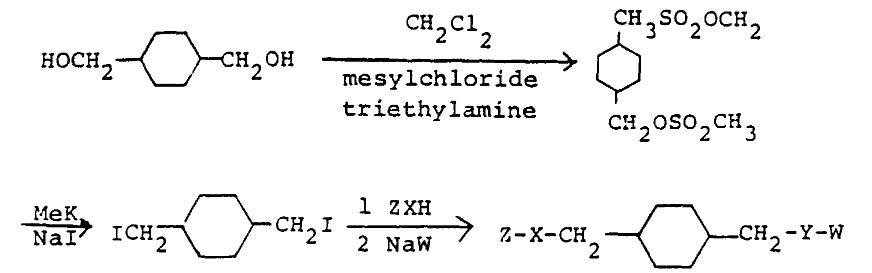 wherein hal is bromine or iodine and Z, X, Y and W are as defined above for formula I.

A compound of formula I or II can be converted to a different compound of formula I or II, respectively, by standard techniques well known in the art. Such conversion techniques are illustrated in the examples.

In general preparing the compounds of this invention involves relatively simple procedures as illustrated by the many examples which appear later in this text.

The following Table I shows the components of the compounds of Formula II illustrated in the Examples:

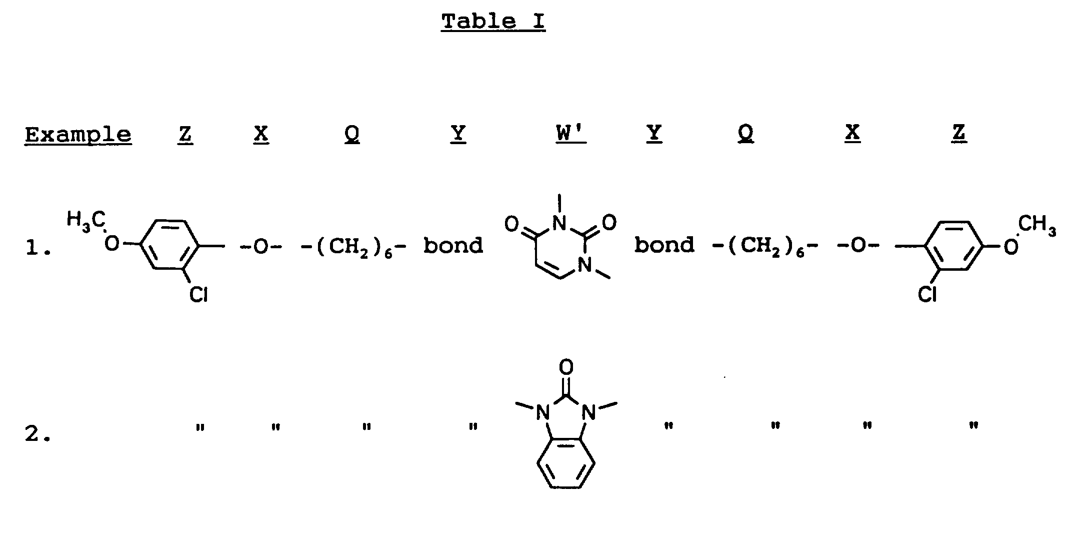

The starting materials for use in the preparation of the compounds of this invention are either commercially available or are prepared by conventional means known in the art.

The following examples illustrate the invention. Fast atom bombardment (FAB) mass spectra (MS) were run on a Finnigan MAT 312 double focussing mass spectrometer, operating at an accelerating voltage of 3 kV. The MS samples were ionized by bombardment with xenon atoms produced by a saddle field ion source from Ion Tech operating with a tube current of 2 mA at an energy of 6 KeV. The proton nuclear magnetic resonance (H¹-NMR) spectra were recorded at 200 MHz on a Varian XL-200 spectronometer; all chemical shift values δ are reported in ppm downfield from tetramethylsilane.

### Example 1

### 1

### 2-{[6-(2-chloro-4-methoxyphenoxy)hexyl]thio} -4,5-dihydrothiazol-4-one

(a) Reflux about 500 mg sodium iodide in 10 ml of acetone with 350 mg. 6-(2-chloro-4-methoxyphenoxy)-hexyl-1-bromide for 5 to 10 minutes, remove the acetone by bubbling nitrogen through the reaction mixture, add methylene chloride, wash with water, then brine and dry over sodium sulfate to obtain 6-(2-chloro-4-methoxy-phenoxy)hexyl-1-iodide.
(b) Add 3 grams of 6-(2-chloro-4-methoxy-phenoxy)hexyl-1-iodide in 10 ml acetonitrile to 1.2 g rhodamine and 20 g cesium carbonate. Stir overnight, then remove the acetonitrile, add methylene chloride and wash with water then brine and dry over sodium sulfate. Elute on a coarse silica column with methylene chloride followed by 5% ethylacetate/methylene chloride and finally 10% ethylacetate/methylene chloride to yield the title compound isolation.

### 2

### 5-{6-(2-chloro-4-methoxyphenoxy) hexyl]thio}-1-methyltetrazole

Reflux 350 mg. 6-(2-chloro-4-methoxyphenoxy) hexyl-1-bromide with 10 ml of acetone and about 500 mg. sodium iodide for five to tem minutes, remove the acetone by bubbling nitrogen through the reaction mixture, add methylene chloride, wash with water, then brine and dry over sodium sulfate to obtain 6-(2-chloro-4-methoxy-phenoxy)hexyl-1-iodide. Add 133 mg of 5-mercapto-1-methyltetrazole, 268 mg. cesium carbonate and 3 ml acetonitrile to the iodide product, stir overnight, add methylene chloride and wash with water, sodium carbonate, water, then brine and dry over sodium sulfate. Remove the solvent and recover the title compound as crystals.

### 3

### 1,3-di-[6-(2-chloro-4-methoxyphenoxy)hexyl] -1,2,3,4-tetrahydropyrimidine-2,4-dione

Stir overnight at room temperature, 300 mg. of the iodide prepared in part 1 or 2, 1.83 gm. 2,4 di-trimethylsilyloxypyrimidine, anhydrous DMF and 1.24 gm. cesium fluoride in a reaction flask. Work up with a water wash in methylene chloride, elute on a coarse silica gel column with methylene chloride followed by 10% ethyl acetate/methylene chloride then 20% ethyl acetate/methylene chloride to isolate the title compound.

### Example 2

### 1-[6-(2-chloro-4-methoxyphenoxy)hexyl]-2-hydroxybenzimidazole and N,N'-bis-[6-(2-chloro-4-methoxyphenoxy)hexyl]-2-benzimidazolone

Stir overnight at room temperature, 1 gm. 2 hydroxybenzimidazole, 2gms. of the iodide prepared in part 1 or 2 of Example 1, 0.35 gm sodium hydroxide and 10 ml DMF in a reaction flask. Partition with water/methylene chloride. Elute on a silica column with 100% methylene chloride, then 50/50 methylene chloride/ethylacetate. Two major fractions are obtained, NMR shows the top spot fraction to be N,N'-bis'-[6-(2-chloro-4-methoxyphenoxy)-hexyl]-2-benzimidazolone, FAB-MS: m/z 615 (M⁺) free base, H¹-NMR-200 mHz; ^{δ} H (CDCl₃), 1.35-1.65 (8H,m), 1.70-1.90 (8H,m), 3.75 (6H,s), 3.80-4.05 (8H,m), 6.7-7.2 (10H,m), and the bottom spot fraction to be 1-[6-(2-chloro-4-methoxyphenoxy)hexyl]-2-hydroxybenzimidazole. FAB-MS: m/z 375 (M⁺); Free base, H¹-NMR-200 mHz; ^{δ}H (CDCl₃), 1.4-1.70 (4H,m), 1.7-1.9 (4H,m), 3.75 (3H,s), 3.85-4.05 (4H,m), 6.7-7.2 (7H,m).

## Claims (Claims for the following Contracting State(s): AT, BE, CH, DE, FR, GB, IT, LI, LU, NL, SE)

1. A compound represented by formula II
Z-X-Q-Y-W'-Y-Q-X-Z II
and pharmaceutically acceptable acid addition, basic addition and quaternary amine salts thereof and pharmaceutically acceptable solvates thereof; wherein
each Z independently is tertiary butyl, phenyl, naphthyl or adamantyl; substituted phenyl, wherein the substituents are one or more of halogen, C₁-C₁₀ alkoxy, phenoxy, nitrile, nitro, phenylsulfonyl, C₁-C₁₀ alkyl-sulfonyl, oxazol-2-yl, C₁-C₁₀ alkanoyl, benzoyl, C₁-C₁₀ alkoxycarbonyl, C₁-C₁₀ alkyl, C₁-C₁₀ alkylthio, phenyl, phenylaminothiocarbonyl, or C₁-C₁₀ alkylaminothiocarbonyl;
X and Y are each independently a bond, or selected from:- 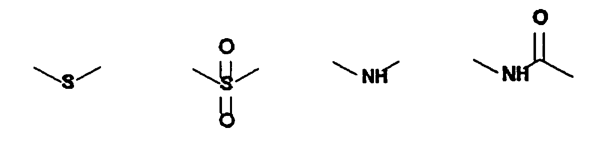 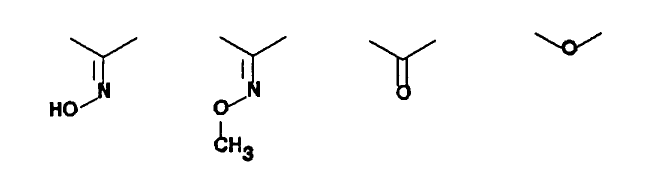
each Q is independently a divalent substituted or unsubstituted, straight or branched chain C₁-C₁₀ alkanediyl, C₁-C₁₀ alkanediyl-C₄-C₇ cycloalkanediyl-C₁-C₁₀ alkanediyl, C₂-C₁₀ alkenediyl, C₂-C₁₀ alkynediyl, phenylene, dihydrofurandiyl, tetrahydrofurandiyl, tetrahydropyrandiyl or, C₁-C₁₀ alkanediyltetrahydrofuranediyl-C₁-C₁₀ alkanediyl, wherein the substituents are one or more of epoxy, fluorine, chlorine, azide, or amino;
W' is a divalent substituted or unsubstituted phenyl or naphthyl group or a heterocyclic single or fused ring containing from 4 to 10 ring atoms, at least one hetero atom of which is a nitrogen atom and the remaining ring atoms being at least one carbon and optionally sulfur or oxygen, wherein the substituents are one or more of hydroxy, oxo, amino, carbamoyl, carboxyl, nitrile, nitro, C₁-C₁₀ alkoxy carbonyl, fluorine, chlorine, iodine, sulfamyl, C₁-C₁₀ alkyl, C₁-C₁₀ alkylthio, C₁-C₁₀ alkoxy, hydroxy C₁-C₁₀ alkyl, C₁-C₁₀ alkoxycarbonyl C₁-C₁₀ alkyl, amino C₁-C₁₀ alkyl, carboxy C₁-C₁₀ alkyl, guanidino, thioureido, C₁-C₁₀ alkylsulfonyl-amino, aminocarbonyl C₁-C₁₀ alkyl, allyloxycarbonylmethyl or carbamoyloxy C₁-C₁₀ alkyl; with the proviso that W' cannot be substituted or unsubstituted isoxazolyl.

2. A compound according to Claim 1 selected from:-
1,3-di[6-(2-chloro-4-methoxyphenoxy)hexyl]-1,2,3,4-tetrahydropyrimidine-2,4-dione; and N,N'-bis-[6-(2-chloro-4-methoxyphenoxy)hexyl]-2-benzimidazolone.

3. A compound as claimed in any previous claim for use as an active therapeutic substance.

4. A pharmaceutical composition comprising a compound according to any of Claims 1-2.

5. Use of a compound according to any of Claims 1-2 in the manufacture of an antiviral medicament.

## Claims (Claims for the following Contracting State(s): ES, GR)

1. A process for producing a compound represented by formula II
Z-X-Q-Y-W'-Y-Q-X-Z II
and pharmaceutically acceptable acid addition, basic addition and quaternary amine salts thereof and pharmaceutically acceptable solvates thereof; wherein
each Z is independently tertiary butyl, phenyl, naphthyl or adamantyl; substituted phenyl, wherein the substituents are one or more of halogen, C₁-C₁₀ alkoxy, phenoxy, nitrile, nitro, phenylsulfonyl, C₁-C₁₀ alkyl-sulfonyl, oxazol-2-yl, C₁-C₁₀ alkanoyl, benzoyl, C₁-C₁₀ alkoxycarbonyl, C₁-C₁₀ alkyl, C₁-C₁₀ alkylthio, phenyl, phenylaminothiocarbonyl, or C₁-C₁₀ alkylaminothiocarbonyl;
X and Y are each independently a bond, or selected from:- 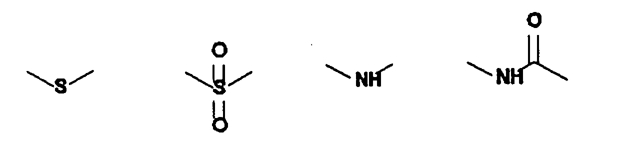 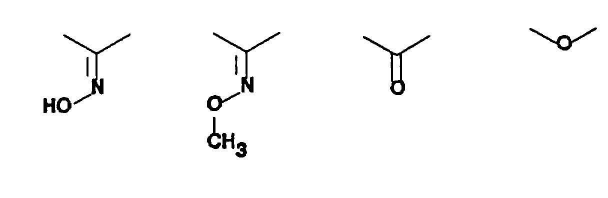
each Q is independently a divalent substituted or unsubstituted, straight or branched chain C₁-C₁₀ alkanediyl, C₁-C₁₀ alkanediyl-C₄-C₇ cycloalkanediyl-C₁-C₁₀-alkanediyl, C₂-C₁₀ alkynediyl, phenylene, dihydrofurandiyl, tetrahydrofurandiyl, tetrahydropyrandiyl or, C₁-C₁₀ alkanediyltetrahydrofuranediyl-C₁-C₁₀ alkanediyl, wherein the substituents are one or more of epoxy, fluorine, chlorine, azide, or amino;
W' is a divalent substituted or unsubstitued phenyl or naphthyl group or a heterocyclic single or fused ring containing from 4 to 10 ring atoms, at least one hetero atom of which is a nitrogen atom and the remaining ring atoms being at least one carbon and optionally sulfur or oxygen, wherein the substituents are one or more of hydroxy, oxo, amino, carbamoyl, carboxyl, nitrile, nitro, C₁-C₁₀ alkoxy carbonyl, fluorine, chlorine, iodine, sulfamyl, C₁-C₁₀ alkyl, C₁-C₁₀ alkylthio, C₁-C₁₀ alkoxy, hydroxy C₁-C₁₀ alkyl, C₁-C₁₀ alkoxycarbonyl C₁-C₁₀ alkyl, amino C₁-C₁₀ alkyl, carboxy C₁-C₁₀ alkyl, guanidino, thioureido, C₁-C₁₀ alkylsulfonyl-amino, aminocarbonyl C₁-C₁₀ alkyl, allyloxycarbonylmethyl or carbamoyloxy C₁-C₁₀ alkyl; with the proviso that W' cannot be substituted or unsubstituted isoxazolyl, characterised by
(A) reacting compounds of the formula
Z-X-Q'-L¹
with a compound of the formula
L³ -Y'-W'''-Y'-L⁴
wherein Z and X are as defined previously, Q' is the same as Q defined previously, or, provided Q is to contain at least one of the groups 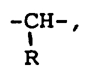 wherein each R is independently hydrogen or C₁-C₁₀ alkyl, Q' may also be the same as Q defined previously minus at least one of the groups 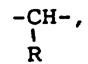
L¹ is a leaving group,
Y' is the same as Y defined previously, or, provided Q is to contain at least one of the groups 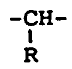 wherein each R is independently hydrogen or C₁-C₁₀ alkyl, Y' may also be the same as Y defined previously plus at least one of the groups 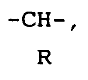
L³ and L⁴ are leaving groups,
W''' is divalent W' as defined above;
wherein any reactive groups are protected if necessary or desired;
the above process followed if necessary or desired by
(i) removal of any protecting groups,
(ii) conversation of a compound so produced to another compound of formula II,
(iii) if more than one compound of formula II is produced, separation of the compounds so produced, or
(iv) conversation of any of the compounds so produced to an acid addition, basic addition, or quaternary amine salt or pharmaceutically acceptable solvate thereof.

2. A process according to Claim 1 for producing: 1,3-di[6-(2-chloro-4-methoxyphenoxy)hexyl]-1,2,3,4-tetrahydropyrimidine-2,4-dione; and N,N'-bis-[6-(2-chloro-4-methoxyphenoxy)hexyl]-2-benzimidazolone.

3. The use of a compound of formula II as defined in any of Claims 1-2 in the manufacture of a medicament.

4. The use of a compound of formula II as defined in any of Claims 1-2 in the manufacture of an antiviral medicament.

## Patentansprüche (Patentansprüche für folgende(n) Vertragsstaat(en): AT, BE, CH, DE, FR, GB, IT, LI, LU, NL, SE)

1. Verbindung, die durch die Formel II
Z-X-Q-Y-W'-Y-Q-X-Z II
dargestellt wird, sowie pharmazeutisch annehmbare Säureadditions-, Basenadditions- und quartäre Ammoniumsalze davon und pharmazeutisch annehmbare Solvate davon, wobei jedes Z unabhängig tertiär-Butyl, Phenyl, Naphthyl oder Adamantyl, substituiertes Phenyl, wobei die Substituenten einer oder mehrere der Reste Halogen, C₁-C₁₀-Alkoxy, Phenoxy, Nitril, Nitro, Phenylsulfonyl, C₁-C₁₀-Alkylsulfonyl, Oxazol-2-yl, C₁-C₁₀-Alkanoyl, Benzoyl, C₁-C₁₀-Alkoxycarbonyl, C₁-C₁₀-Alkyl, C₁-C₁₀-Alkylthio, Phenyl, Phenylaminothiocarbonyl oder C₁-C₁₀-Alkylaminothiocarbonyl sind, ist,
X und Y jeweils unabhängig eine Bindung oder aus: 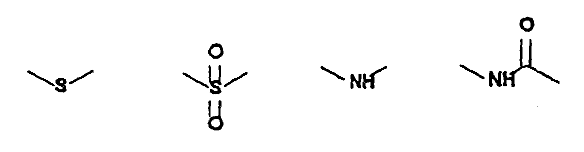 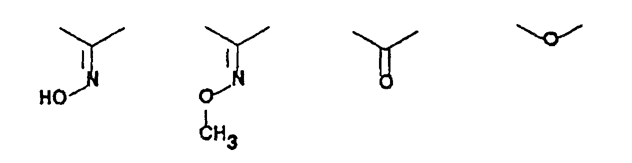 ausgewählt sind,
jedes Q unabhängig ein zweiwertiges substituiertes oder unsubstituiertes geradkettiges oder verzweigtes C₁-C₁₀-Alkandiyl, C₁-C₁₀-Alkandiyl-C₄-C₇-cycloalkandiyl-C₁-C₁₀-alkandiyl, C₂-C₁₀-Alkendiyl, C₂-C₁₀-Alkindiyl, Phenylen, Dihydrofurandiyl, Tetrahydrofurandiyl, Tetrahydropyrandiyl oder C₁-C₁₀-Alkandiyltetrahydrofurandiyl-C₁-C₁₀-alkandiyl ist, wobei die Substituenten einer oder mehrere der Reste Epoxy, Fluor, Chlor, Azid oder Amino sind,
W' eine zweiwertige substituierte oder unsubstituierte Phenyl- oder Naphthylgruppe oder ein heterocyclischer einfacher oder kondensierter Ring mit 4 bis 10 Ringatomen ist, wobei wenigstens eines der Heteroatome ein Stickstoffatom ist und es sich bei den übrigen Ringatomen um wenigstens ein Kohlenstoffatom und gegebenenfalls Schwefel oder Sauerstoff handelt, wobei die Substituenten einer oder mehrere der Reste Hydroxy, Oxo, Amino, Carbamoyl, Carboxyl, Nitril, Nitro, C₁-C₁₀-Alkoxycarbonyl, Fluor, Chlor, Iod, Sulfamyl, C₁-C₁₀-Alkyl, C₁-C₁₀-Alkylthio, C₁-C₁₀-Alkoxy, Hydroxy-C₁-C₁₀-alkyl, C₁-C₁₀-Alkoxycarbonyl-C₁-C₁₀-alkyl, Amino-C₁-C₁₀-alkyl, Carboxy-C₁-C₁₀-alkyl, Guanidino, Thioureido, C₁-C₁₀-Alkylsulfonylamino, Aminocarbonyl-C₁-C₁₀-alkyl, Allyloxycarbonylmethyl oder Carbamoyloxy-C₁-C₁₀-alkyl sind, mit der Maßgabe, daß W' nicht substituiertes oder unsubstituiertes Isoxazolyl sein kann.

2. Verbindung gemäß Anspruch 1, die aus 1,3-Di[6-(2-chlor-4-methoxyphenoxy)hexyl]-1,2,3,4-tetrahydropyrimidin-2,4-dion und N,N'-Bis[6-(2-chlor-4-methoxyphenoxy)hexyl]-2-benzimidazolon ausgewählt ist.

3. Verbindung gemäß einem der vorhergehenden Ansprüche zur Verwendung als therapeutischer Wirkstoff.

4. Pharmazeutische Zusammensetzung, die eine Verbindung gemäß einem der Ansprüche 1-2 umfaßt.

5. Verwendung einer Verbindung gemäß einem der Ansprüche 1-2 bei der Herstellung eines antiviralen Medikaments.

## Patentansprüche (Patentansprüche für folgende(n) Vertragsstaat(en): ES, GR)

1. Verfahren zur Herstellung einer Verbindung, die durch die Formel II
Z-X-Q-Y-W'-Y-Q-X-Z II
dargestellt wird, sowie pharmazeutisch annehmbarer Säureadditions-, Basenadditions- und quartärer Ammoniumsalze davon und pharmazeutisch annehmbarer Solvate davon, wobei jedes Z unabhängig tertiär-Butyl, Phenyl, Naphthyl oder Adamantyl, substituiertes Phenyl, wobei die Substituenten einer oder mehrere der Reste Halogen, C₁-C₁₀-Alkoxy, Phenoxy, Nitril, Nitro, Phenylsulfonyl, C₁-C₁₀-Alkylsulfonyl, Oxazol-2-yl, C₁-C₁₀-Alkanoyl, Benzoyl, C₁-C₁₀-Alkoxycarbonyl, C₁-C₁₀-Alkyl, C₁-C₁₀-Alkylthio, Phenyl, Phenylaminothiocarbonyl oder C₁-C₁₀-Alkylaminothiocarbonyl sind, ist,
X und Y jeweils unabhängig eine Bindung oder aus: 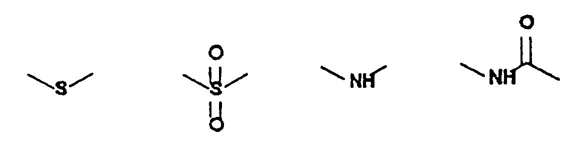 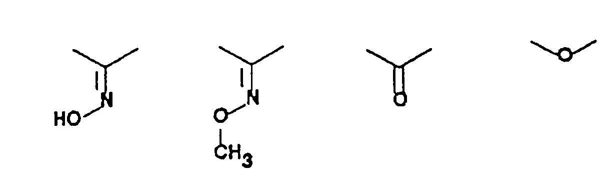 ausgewählt sind,
jedes Q unabhängig ein zweiwertiges substituiertes oder unsubstituiertes geradkettiges oder verzweigtes C₁-C₁₀-Alkandiyl, C₁-C₁₀-Alkandiyl-C₄-C₇-cycloalkandiyl-C₁-C₁₀-alkandiyl, C₂-C₁₀-Alkindiyl, Phenylen, Dihydrofurandiyl, Tetrahydrofurandiyl, Tetrahydropyrandiyl oder C₁-C₁₀-Alkandiyltetrahydrofurandiyl-C₁-C₁₀-alkandiyl ist, wobei die Substituenten einer oder mehrere der Reste Epoxy, Fluor, Chlor, Azid oder Amino sind,
W' eine zweiwertige substituierte oder unsubstituierte Phenyl- oder Naphthylgruppe oder ein heterocyclischer einfacher oder kondensierter Ring mit 4 bis 10 Ringatomen ist, wobei wenigstens eines der Heteroatome ein Stickstoffatom ist und es sich bei den übrigen Ringatomen um wenigstens ein Kohlenstoffatom und gegebenenfalls Schwefel oder Sauerstoff handelt, wobei die Substituenten einer oder mehrere der Reste Hydroxy, Oxo, Amino, Carbamoyl, Carboxyl, Nitril, Nitro, C₁-C₁₀-Alkoxycarbonyl, Fluor, Chlor, Iod, Sulfamyl, C₁-C₁₀-Alkyl, C₁-C₁₀-Alkylthio, C₁-C₁₀-Alkoxy, Hydroxy-C₁-C₁₀-alkyl, C₁-C₁₀-Alkoxycarbonyl-C₁-C₁₀-alkyl, Amino-C₁-C₁₀-alkyl, Carboxy-C₁-C₁₀-alkyl, Guanidino, Thioureido, C₁-C₁₀-Alkylsulfonylamino, Aminocarbonyl-C₁-C₁₀-alkyl, Allyloxycarbonylmethyl oder Carbamoyloxy-C₁-C₁₀-alkyl sind, mit der Maßgabe, daß W' nicht substituiertes oder unsubstituiertes Isoxazolyl sein kann, gekennzeichnet durch
(A) Umsetzen von Verbindungen der Formel
Z-X-Q'-L¹
mit einer Verbindung der Formel
L³-Y'-W'''-Y'-L⁴,
wobei Z und X wie zuvor definiert sind, Q' dasselbe wie das zuvor definierte Q ist oder, falls Q wenigstens eine der Gruppen 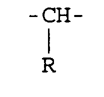 enthalten soll, wobei jedes R unabhängig Wasserstoff oder C₁-C₁₀-Alkyl ist, Q' ebenfalls dasselbe wie das zuvor definierte Q minus wenigstens einer der Gruppen 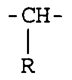 sein kann,
L¹ eine Abgangsgruppe ist,
Y' dasselbe wie das zuvor definierte Y ist oder, falls Q wenigstens eine der Gruppen 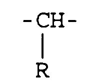 enthalten soll, wobei jedes R unabhängig Wasserstoff oder C₁-C₁₀-Alkyl ist, Y' ebenfalls dasselbe wie das zuvor definierte Y plus wenigstens eine der Gruppen 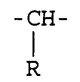 sein kann,
L³ und L⁴ Abgangsgruppen sind,
W''' zweiwertiges W' ist, wie es oben definiert ist,
wobei jede reaktive Gruppe, falls erforderlich oder erwünscht, geschützt ist;
und anschließend an das obige Verfahren, falls erforderlich oder erwünscht,
(i) Entfernung der Schutzgruppen,
(ii) Umwandlung einer so hergestellten Verbindung in eine andere Verbindung der Formel II,
(iii) falls mehr als eine Verbindung der Formel II hergestellt wird, Trennung der so hergestellten Verbindungen oder
(iv) Umwandlung einer oder mehrerer der so hergestellten Verbindungen in ein Säureadditions-, Basenadditionsoder quartäres Ammoniumsalz oder pharmazeutisch annehmbares Solvat davon.

2. Verfahren gemäß Anspruch 1 zur Herstellung von 1,3-Di[6-(2-chlor-4-methoxyphenoxy)hexyl]-1,2,3,4-tetrahydropyrimidin-2,4-dion oder N,N'-Bis [6-(2-chlor-4-methoxyphenoxy)-hexyl]-2-benzimidazolon.

3. Verwendung einer Verbindung der Formel II, wie sie in einem der Ansprüche 1-2 definiert ist, bei der Herstellung eines Medikaments.

4. Verwendung einer Verbindung der Formel II, wie sie in einem der Ansprüche 1-2 definiert ist, bei der Herstellung eines antiviralen Medikaments.

## Revendications (Revendications pour l'(les) Etat(s) contractant(s) suivant(s): AT, BE, CH, DE, FR, GB, IT, LI, LU, NL, SE)

1. Composé représenté par la formule II
Z-X-Q-Y-W'-Y-Q-X-Z II
et ses sels d'addition d'acide, d'addition basique et d'amine quaternaire pharmaceutiquement acceptables ainsi que ses solvates pharmaceutiquement acceptables; où
chaque Z est indépendamment butyle tertiaire, phényle, naphtyle ou adamantyle; phényle substitué, où les substituants sont un ou plusieurs parmi halogène, alcoxy C₁-C₁₀, phénoxy, nitrile, nitro, phénylsulfonyle, alkyl-sulfonyle C₁-C₁₀, oxazol-2-yle, alcanoyle C₁-C₁₀, benzoyle, alcoxycarbonyle C₁-C₁₀, alkyle C₁-C₁₀, alkylthio C₁-C₁₀, phényle, phénylaminothiocarbonyle, ou alkylaminothiocarbonyle C₁-C₁₀ ;
chacun de X et Y est indépendamment une liaison ou bien chacun est choisi parmi : 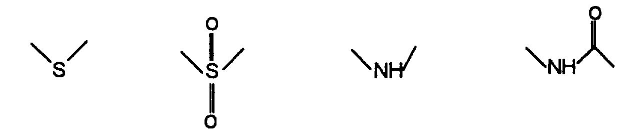 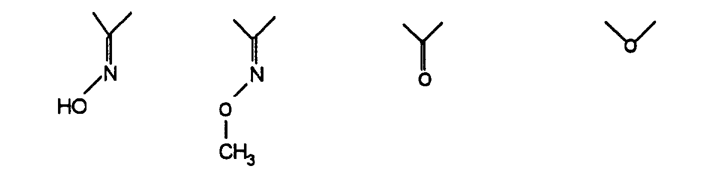
chaque Q est indépendamment un alcanediyle C₁-C₁₀, alcènediyle C₁-C₁₀-cycloalcanediyle C₄-C₇-alcanediyle C₁-C₁₀ alcanediyle C₂-C₁₀, alcynediyle C₂-C₁₀, phénylène, dihydrofuranediyle, tétrahydrofuranediyle, tétrahydropyranediyle ou alcanediyltétrahydrofuranediyle C₁-C₁₀-alcanediyle C₁-C₁₀, où les substituants sont un ou plusieurs parmi époxy, fluor, chlore, azide, ou amino;
W' est un groupe phényle ou naphtyle substitué ou non substitué divalent ou bien un cycle hétérocyclique individuel ou fusionné contenant de 4 à 10 atomes dans le cycle, dont au moins un hétéroatome est un atome d'azote et dont les atomes restants dans le cycle sont au moins un carbone et facultativement du soufre ou, de l'oxygène, où les substituants sont un ou plusieurs parmi hydroxy, oxo, amino, carbamoyle, carboxyle, nitrile, nitro, alcoxy carbonyle C₁-C₁₀, fluor, chlore, iode, sulfamyle, alkyle C₁-C₁₀, alkylthio C₁-C₁₀, alcoxy C₁-C₁₀, hydroxy alkyle C₁-C₁₀, alcoxycarbonyle C₁-C₁₀-alkyle C₁-C₁₀, aminoalkyle C₁-C₁₀, carboxyalkyle C₁-C₁₀, guanidino, thiouréido, alkylsulfonylamino C₁-C₁₀, aminocarbonyl alkyle C₁-C₁₀, allyloxycarbonylméthyle ou carbamoyloxy alkyle C₁-C₁₀; à condition que W' ne puisse être isoxazolyle substitué ou non substitué.

2. Composition selon la revendication 1 sélectionné parmi :
1,3-di[6-(2-chloro-4-méthoxyphénoxy)hexyl]-1,2,3,4-tétrahydropyrimidine-2,4-dione; et N,N'-bis-[6-(2-chloro-4-méthoxyphénoxy)hexyl]-2-benzimidazolone.

3. Composé selon l'une quelconque des revendications précédentes pour une utilisation comme substance thérapeutique active.

4. Composition pharmaceutique comprenant un composé selon l'une quelconque des revendications 1-2.

5. Utilisation d'un composé selon l'une quelconque des revendications 1-2 dans la fabrication d'un médicament antiviral.

## Revendications (Revendications pour l'(les) Etat(s) contractant(s) suivant(s): ES, GR)

1. Procédé de production d'un composé représenté par la formule II
Z-X-Q-Y-W'-Y-Q-X-Z II
et ses sels d'addition d'acide, d'addition basique et d'amine quaternaire pharmaceutiquement acceptables ainsi que ses solvates pharmaceutiquement acceptables; où
chaque Z est indépendamment butyle tertiaire, phényle, naphtyle ou adamantyle; phényle substitué, où les substituants sont un ou plusieurs parmi halogène, alcoxy C₁-C₁₀, phénoxy, nitrile, nitro, phénylsulfonyle, alkyl-sulfonyle C₁-C₁₀, oxazol-2-yle, alcanoyle C₁-C₁₀, benzoyle, alcoxycarbonyle C₁-C₁₀, alkyle C₁-C₁₀, alkylthio C₁-C₁₀, phényle, phénylaminothiocarbonyle, ou alkylaminothiocarbonyle C₁-C₁₀;
chacun de X et Y est indépendamment une liaison ou bien chacun est choisi parmi : 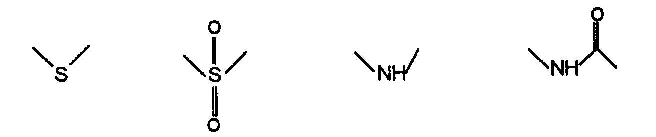 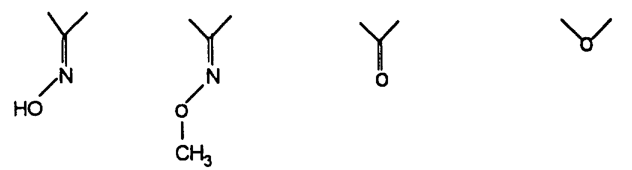 chaque Q est indépendamment un alcanediyle C₁-C₁₀, alcènediyle C₁-C₁₀-cycloalcanediyle C₄-C₇-alcanediyle C₁-C₁₀, alcanediyle C₂-C₁₀, alcynediyle C₂-C₁₀, phénylène, dihydrofuranediyle, tétrahydrofuranediyle, tétrahydropyranediyle ou alcanediytétrahydrofuranediyle C₁-C₁₀-alcanediyle C₁-C₁₀, où les substituants sont un ou plusieurs parmi époxy, fluor, chlore, azide, ou amino;
W' est un groupe phényle ou naphtyle substitué ou non substitué divalent ou bien un cycle hétérocyclique individuel ou fusionné contenant de 4 à 10 atomes dans le cycle, dont au moins un hétéroatome est un atome d'azote et dont les atomes restants dans le cycle sont au moins un carbone et facultativement du soufre ou, de l'oxygène, où les substituants sont un ou plusieurs parmi hydroxy, oxo, amino, carbamoyle, carboxyle, nitrile, nitro, alcoxy carbonyle C₁-C₁₀, fluor, chlore, iode, sulfamyle, alkyle C₁-C₁₀, alkylthio C₁-C₁₀, alcoxy C₁-C₁₀, hydroxy alkyle C₁-C₁₀ alcoxycarbonile C₁-C₁₀-aCkySe C₁-C₁₀, aminoalkyle C₁-C₁₀, carboxyalkyle C₁-C₁₀, guanidino, thiouréido, alkylsulfonylamino C₁-C₁₀, aminocarbonyl alkyle C₁-C₁₀, allyloxycarbonylméthyle ou carbamoyloxy alkyle C₁-C₁₀; à condition que W' ne puisse être isoxazolyle substitué ou non substitué, caractérisé par
(A) la réaction de composés de la formule
Z-X-Q'-L'
avec un composé de la formule
L³-Y'-W'''-Y'-L⁴
où Z et X sont tels que définis précédemment, Q' est le même que Q précédemment défini ou bien à condition que Q doive contenir au moins l'un des groupes 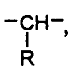 où chaque R est indépendamment hydrogène ou alkyle C₁-C₁₀, Q' peut également être le même que Q défini précédemment moins au moins l'un des groupes 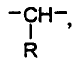
L¹ est un groupe partant,
Y' est le même que Y précédemment défini, ou bien, à condition que Q doive contenir au moins l'un des groupes 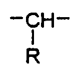 où chaque R est indépendamment hydrogène ou alkyle C₁-C₁₀, Y' peut égalment être tel que défini précédemment plus au moins l'un des groupes 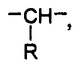
L³ et L⁴ sont des groupes partants,
W''' est W' divalent tel que défini ci-dessus;
où tous les groupes réactifs sont protégés si nécessaire ou si on le souhaite;
le procédé ci-dessus suivi si nécessaire ou si on le souhaite par
(i) élimination de tous les groupes protecteurs,
(ii) conversion d'un composé ainsi produit en un autre composé de formule II,
(iii) si plus d'un composé de formule II est produit, séparation des composés ainsi produits, ou
(iv) conversion de tous les composés ainsi produits en un sel d'addition d'acide, d'addition basique, ou d'amine quaternaire ou solvate pharmaceutiquement acceptable.

2. Procédé selon la revendication 1 pour la production de la 1,3-di[6-(2-chloro-4 méthoxyphénoxy)hexyl]-1,2,3,4-tétrahydropyrimidine-2,4-dione; et de la N,N'-bis-[6-(2-chloro-4-méthoxyphénoxy)hexyl]-2-benzimidazolone.

3. Utilisation d'un composé de formule II tel que défini selon l'une quelconque des revendications 1-2 dans la fabrication d'un médicament.

4. Utilisation d'un composé de formule II tel que défini selon l'une quelconque des revendications 1-2 dans la fabrication d'un médicament antiviral.
